# FASCICULE DE BREVET EUROPEEN

(11) **EP 2 931 189 B1**
(45) Date de publication et mention de la délivrance du brevet: **24.05.2017**
(21) Numéro de dépôt: 13818299.3
(22) Date de dépôt: 13.12.2013
(51) Int. Cl.: A61F 5/01

(54) **DISPOSITIF ORTHOPÉDIQUE POUR UN MEMBRE INFÉRIEUR HUMAIN, CHAUSSURE ET PROTHÈSE ÉQUIPÉES D'UN TEL DISPOSITIF**
ORTHOPÄDISCHE VORRICHTUNG FÜR EINE UNTERE EXTREMITÄT EINES MENSCHEN, SCHUHWERK UND PROTHESE MIT SOLCH EINER VORRICHTUNG
ORTHOPAEDIC DEVICE FOR A LOWER LIMB OF A HUMAN, FOOTWEAR AND PROSTHESIS PROVIDED WITH SUCH A DEVICE

(30) Priorité: 14.12.2012 FR 1262103
(43) Date de publication de la demande: 21.10.2015
(73) Titulaire: Caussé, Benoît, 31820 Pibrac (FR)
(72) Inventeur: Caussé, Benoît, 31820 Pibrac (FR)
(74) Mandataire: Cabinet BARRE LAFORGUE & associés
(86) Numéro de dépôt international: PCT/FR2013/053073
(87) Numéro de publication internationale: WO 2014/091171

(56) Documents cités:
- DE-C- 95 326
- DE-U1- 8 534 733
- US-A- 3 680 549
- US-A- 4 637 381
- US-A- 5 658 241
- US-A- 5 718 673
- US-A1- 2010 324 461

## Description

L'invention concerne un dispositif orthopédique pour un membre inférieur humain. L'invention concerne également un tel dispositif orthopédique comprenant en outre une partie jambière. L'invention concerne également une chaussure équipée d'un tel dispositif orthopédique ainsi qu'une prothèse équipée d'un tel dispositif orthopédique.

Un dispositif orthopédique selon l'invention est utile pour des personnes présentant une difficulté à la marche, par exemple des personnes atteintes d'affections neurologiques centrales et périphériques telles que les paralysies de la jambe et du pied, ou encore des personnes atteintes par certaines myopathies. Un dispositif orthopédique selon l'invention est également utile pour des personnes présentant des atteintes articulaires traumatiques ou non, ou certaines amputations.

Dans tout le texte, on entend par "dispositif orthopédique", tout dispositif destiné à soutenir un membre inférieur humain et/ou à pallier les déficiences fonctionnelles d'un membre inférieur humain ou, éventuellement, tout dispositif destiné à remplacer au moins partiellement un membre inférieur humain.

La marche est un mécanisme complexe mettant en jeu de nombreux muscles de l'ensemble du corps humain. Dans ce mécanisme, les membres inférieurs doivent assurer à la fois une stabilisation et une propulsion du corps.

Le cycle de marche de chaque membre inférieur humain comprend une phase d'appui et une phase oscillante. La phase d'appui comprend toutes les étapes au cours desquelles le pied est en contact avec le sol. La phase oscillante comprend les étapes au cours desquelles le pied n'est pas en contact avec le sol, permettant l'avancée du membre inférieur.

A l'attaque du pas lors de la phase oscillante, la jambe se déplace, en suspension, vers l'avant par rapport à l'autre jambe, jusqu'à ce que le talon entre en contact avec le sol, prenant ainsi un premier appui. La phase d'appui comprend une première étape d'appui du talon au sol (contact taligrade), une deuxième étape d'appui de la plante du pied (appui plantigrade) puis une dernière étape d'appui de l'avant-pied et des orteils, en particulier du gros orteil (appui digitigrade). Ainsi, en fin de pas, la perte de contact du pied avec le sol a lieu physiologiquement au niveau du gros orteil.

Le steppage est un des troubles les plus fréquents de la marche et dont les causes peuvent être très diverses. Il peut se rencontrer de façon constitutionnelle, dans le cas de maladies acquises ou congénitales et dans le cadre de pathologies telles que les myopathies, les maladies de Charcot-Marie-Tooth, le syndrome de Guillain-Barré, la sclérose en plaques, les hémiplégies d'origine centrale par accident vasculaire cérébral ou à la suite de divers traumatismes accidentels. Il consiste en un défaut des muscles releveurs du pied affectant ou empêchant la dorsiflexion (mouvement de flexion du pied vers le haut du corps). Ainsi, une personne dont le pied est tombant ou ballant est contrainte de lever exagérément la jambe à l'attaque du pas de façon à éviter que son avant-pied ne touche le sol au cours de la phase oscillante de la marche.

Pour remédier à ces problèmes, on connait par exemple (US 6 083 184) une orthèse de cheville formant une gouttière de réception du pied. Une telle orthèse de cheville comprend une semelle s'étendant sous le pied, de la cheville aux orteils, une partie entourant la cheville et une zone de fixation de l'orthèse sur la partie supérieure de la cheville. Une telle orthèse de cheville ne permet pas un accompagnement dynamique des mouvements lors de la marche, son rôle étant purement statique. Une orthèse selon le préambule de la revendication 1 annexée est connue de US 2010/0324461. FR 2 953 126 propose une orthèse dynamique comprenant une semelle de pied et un bracelet de jambe reliés par une structure comportant un vérin élastique à l'arrière de la jambe et du pied. L'actionnement du vérin permet à la semelle de pivoter autour de l'articulation de la cheville de façon à relever le pied en fin de pas.

Une telle orthèse dynamique nécessite l'adjonction de dispositifs additionnels complexes et requiert des réglages réguliers de la part du personnel médical après sa pose sur un patient. En outre, une telle orthèse dynamique ne permet pas d'accompagner de façon satisfaisante et suffisamment dynamique les mouvements lors de la marche, ni de procurer un effet propulseur physiologique et spécifique à la bipédie humaine en fin de pas.

L'invention vise donc à pallier ces inconvénients et à proposer un dispositif orthopédique pour un membre inférieur humain permettant de :
- pallier les déficiences des muscles releveurs du pied,
- procurer un effet releveur à l'attaque du pas lors de la marche,
- procurer un effet propulseur en fin de pas lors de la marche, ou éventuellement de la course à pied,
- procurer un effet stabilisateur, et en particulier un effet de stabilisation latérale, d'un membre inférieur humain, notamment au cours des différentes phases de la marche ou de la course à pied,
- maintenir le pied dans une position normale et adaptée à la marche,
- favoriser le rétablissement musculaire par une stimulation spécifique et propre des muscles des membres inférieurs humain, en particulier après un accident ou en cas de maladie affectant les muscles des membres inférieurs, et notamment des muscles releveurs du pied, et
- être porté si besoin, dans une chaussure, notamment une chaussure de sport, une chaussure de ville ou une chaussure orthopédique et en particulier dans une chaussure maintenant la cheville.

L'invention vise également à proposer un dispositif orthopédique ayant pour avantage d'être peu invasif et ergonomique (léger et peu encombrant) pour le patient.

Pour ce faire, l'invention concerne un dispositif orthopédique pour un membre inférieur humain comprenant :
- un segment antérieur adapté pour recevoir la plante d'un avant-pied d'un pied,
- un segment postérieur adapté pour recevoir la face inférieure du talon dudit pied, dans lequel :
   - le segment antérieur et le segment postérieur sont formés de deux pièces distinctes l'une de l'autre,
   - le segment antérieur et le segment postérieur sont reliés par au moins un arceau supérieur:
      ∘ s'étendant au-dessus du segment antérieur et du segment postérieur en ménageant un logement de réception du pied,
      ∘ adapté pour maintenir le segment antérieur et le segment postérieur dans une position de repos, caractérisé en ce que :
         l'arceau supérieur est élastique et adapté pour permettre un déplacement relatif du segment antérieur et du segment postérieur l'un par rapport à l'autre, à partir de la position de repos, et pour rappeler élastiquement le segment antérieur et le segment postérieur en position de repos.

Un dispositif orthopédique selon l'invention est adapté pour accueillir un pied, l'avant-pied et le talon du pied reposant respectivement sur le segment antérieur et le segment postérieur du dispositif, ces derniers étant maintenus et reliés élastiquement par un arceau supérieur.

Le segment antérieur et le segment postérieur sont formés de deux pièces distinctes l'une de l'autre, c'est-à-dire de deux segments distincts qui ne sont pas formés d'un même segment continu ni reliés l'un à l'autre dans un même plan (en position de repos), à la différence d'une semelle continue s'étendant sous toute la surface inférieure de la voûte plantaire. En d'autres termes, le segment antérieur et le segment postérieur sont formés de deux pièces distinctes et distantes l'une de l'autre selon une direction longitudinale du pied et distinctes et distantes l'une de l'autre dans un plan comprenant une direction longitudinale du pied et perpendiculaire à un plan sagittal (c'est-à-dire un plan parallèle à la surface inférieure de la voûte plantaire).

Un dispositif orthopédique selon l'invention consiste en particulier en une orthèse pour un membre inférieur humain. Le dispositif orthopédique selon l'invention peut également constituer dans certains cas une prothèse d'au moins une partie d'un membre inférieur humain, l'orthèse étant alors par exemple associée à une prothèse de pied et/ou de jambe.

Dans tout le texte, on entend par "avant-pied" la partie antérieure d'un pied comprenant les orteils et les cinq os métatarsiens.

Un dispositif orthopédique selon l'invention permet de maintenir le pied dans une position anatomiquement adaptée en position de repos, par exemple lors de la station debout, ainsi qu'au cours de tout autre mouvement du membre inférieur, notamment les mouvements associés à la marche ou à la course à pied, sans les empêcher. Un dispositif orthopédique selon l'invention est ainsi adapté pour accompagner le pied dans ses mouvements tout en exerçant une force de rappel vers la position de repos des segments antérieur et postérieur, grâce à l'arceau supérieur.

L'inventeur a constaté avec surprise que contrairement notamment à une semelle en forme de plaque flexible, un dispositif orthopédique selon l'invention procure non seulement un maintien du pied dans une position anatomiquement correcte, en particulier en position de repos, notamment en position statique, par exemple lors de la station debout, mais également un effet dynamique propulseur en fin de pas, à la façon d'un ressort ou d'un arc élastique restituant de l'énergie cinétique (sous la forme d'une projection du pied au début de la phase oscillante) après avoir emmagasiné de l'énergie potentielle élastique (par effet du poids du corps en appui sur un pied).

L'inventeur a également constaté avec surprise qu'un dispositif orthopédique selon l'invention permet de pallier au déficit des muscles des membres inférieurs humains et en particulier du muscle pédieux (ou muscle court extenseur des orteils) dont le rôle semblerait être primordial au mécanisme de la marche. En effet, l'inventeur a pu constater une amélioration importante du fonctionnement du muscle pédieux, celui-ci semblant jouer le rôle d'un capteur de tension actif régulant les autres muscles entourant la cheville.

Ce mécanisme de restitution d'énergie permettant de restituer ou d'améliorer l'impulsion vers un nouveau pas en fin de pas réside dans la présence d'au moins deux segments distincts, le segment antérieur et le segment postérieur, reliés élastiquement par l'arceau supérieur.

En outre, l'inventeur a pu constater qu'un dispositif orthopédique selon l'invention est particulièrement efficace en tant qu'outil rééducatif après un traumatisme ou une chirurgie quand le muscle pédieux est insuffisant ou inactif fonctionnellement. Le dispositif orthopédique selon l'invention peut être utilisé dans les cas d'entorses de la cheville, de tendinites ou de ruptures tendineuses (en particulier du tendon d'Achille), d'algodystrophies, et dans les suites d'intervention chirurgicales sur le pied et le membre inférieur. Le dispositif orthopédique selon l'invention permet de procurer une impulsion devenue insuffisante dans de tels cas et ainsi d'harmoniser le pas et son déroulé avec un schéma moteur non pas statique mais dynamique.

Il est à noter que le segment antérieur est formé d'une première pièce et le segment postérieur est formé d'une deuxième pièce, ladite première pièce et ladite deuxième pièce étant distinctes l'une de l'autre, c'est-à-dire distinctes et distantes l'une de l'autre dans un plan parallèle à la surface inférieure de la voûte plantaire. Un dispositif orthopédique selon l'invention ne présente donc pas une semelle continue s'étendant sous toute la surface inférieure de la voûte plantaire.

Les segments antérieur et postérieur peuvent présenter toute forme adaptée pour soutenir respectivement la plante de l'avant-pied et le talon. En particulier, avantageusement et selon l'invention, le segment antérieur est formé d'une première plaque et le segment postérieur est formé d'une deuxième plaque, distincte (et distante) de la première plaque.

Le segment postérieur étant adapté pour recevoir le talon et être disposé sous ce dernier, et le segment antérieur étant adapté pour recevoir l'avant-pied et être disposé sous ce dernier, les segments postérieur et antérieur sont disposés dans le prolongement longitudinal (et en particulier sagittal) l'un de l'autre. En effet, avantageusement et selon l'invention, le segment antérieur et le segment postérieur s'étendent dans le prolongement longitudinal l'un de l'autre et sont longitudinalement écartés l'un de l'autre, au moins en position de repos.

Avantageusement et selon l'invention, outre au moins un arceau supérieur, le segment antérieur et le segment postérieur ne sont pas solidaires ni reliés de façon continue l'un à l'autre dans un plan récepteur de la face inférieure du pied à la manière d'une semelle. Au contraire, avantageusement et selon l'invention, le segment antérieur et le segment postérieur sont reliés élastiquement l'un à l'autre exclusivement par au moins un arceau supérieur au-dessus du pied.

Ainsi, des déplacements du segment antérieur et du segment postérieur l'un par rapport à l'autre sont permis en particulier à la fois globalement selon la direction longitudinale du pied (qui est aussi une direction sagittale) et selon une direction orthogonale à la direction longitudinale du pied, dans le plan sagittal. Les déplacements de l'avant-pied et du talon d'un pied équipé d'un dispositif orthopédique selon l'invention, notamment lors de la marche ou de la course à pied, sont donc autorisés.

En effet, avantageusement et selon l'invention, chaque arceau supérieur est adapté pour permettre un déplacement relatif du segment antérieur et du segment postérieur l'un par rapport à l'autre, à partir de la position de repos dans un plan sagittal, et globalement selon la direction longitudinale du pied.

A chaque déplacement relatif du segment antérieur et/ou du segment postérieur par rapport à la position de repos, le(les) arceau(x) supérieur(s) exerce(nt) une force de rappel du segment antérieur et/ou du segment postérieur en position de repos.

Chaque arceau supérieur s'étend au-dessus du segment antérieur et du segment postérieur et de façon à pouvoir les relier élastiquement et à les maintenir en position de repos ou à les rappeler vers la position de repos après avoir été déplacés par les efforts exercés sur le segment antérieur et le segment postérieur par les pieds, les efforts dynamiques de la marche, et le poids du corps.

Plusieurs modes de réalisation de l'invention sont possibles en ce qui concerne le nombre, la forme, les dimensions, les matières, les dispositions relatives du (des) arceau(x) supérieur(s). Avantageusement, un dispositif orthopédique selon l'invention comprend un arceau supérieur qui s'étend obliquement et au contact de la face supérieure du pied, entre une extrémité latérale du segment antérieur et une extrémité latérale du segment postérieur. En particulier, l'arceau supérieur s'étend entre une extrémité latérale du segment antérieur et une extrémité latérale du segment postérieur de façon à s'enrouler autour et en contact du pied, à la façon d'une portion de bandage hélicoïdal ou d'une bande enroulée sur elle-même. Plus particulièrement, chaque arceau est adapté pour passer au-dessus et à proximité du cou-de-pied, notamment au niveau de la base des cinq os métatarsiens et/ou des cinq os métatarsiens.

Dans une variante de réalisation particulièrement avantageuse d'un dispositif orthopédique selon l'invention, l'arceau supérieur s'étend entre une extrémité latérale externe du segment antérieur et une extrémité latérale interne du segment postérieur. De cette façon, le dispositif orthopédique forme une structure continue depuis l'extrémité interne du segment antérieur jusqu'au rebord latéral externe du segment postérieur.

Le segment antérieur est adapté pour recevoir au moins une partie de la face inférieure de l'avant-pied, et être disposé en regard et en dessous d'au moins une partie de la face inférieure de l'avant-pied. Le segment antérieur s'étend ainsi sous au moins un métatarse du pied de façon à soutenir au moins partiellement la face inférieure de l'avant-pied et à pouvoir exercer une force de rappel de l'avant-pied vers la position de repos lors d'un déplacement relatif des segments hors de la position de repos.

En particulier, avantageusement et selon l'invention, le segment antérieur est adapté pour recevoir le gros orteil du pied. Le segment antérieur est donc adapté pour être disposé notamment en regard et en dessous de la face inférieure du gros orteil du pied. Lors de la station debout ou des phases d'appui de la marche ou de la course à pied, le gros orteil repose donc sur le segment antérieur d'un dispositif orthopédique selon l'invention. Ainsi, lors de la marche, quand le pied est fléchi en appui au sol sur le gros orteil, le talon soulevé par rapport au sol, l'arceau supérieur élastique permet de propulser dynamiquement le pied vers l'avant, c'est-à-dire de passer en phase oscillante.

Dans une variante de réalisation avantageuse d'un dispositif orthopédique selon l'invention, le segment antérieur s'étend transversalement entre un bord latéral interne adapté pour recevoir le gros orteil et un bord latéral externe adapté pour recevoir le cinquième métatarse du pied, en particulier la région située à proximité du petit orteil et notamment la région proche de la tête du cinquième métatarsien (avant la phalange du petit orteil). Le segment antérieur est ainsi disposé de façon à s'étendre sous toute la largeur du pied et à exercer un soutien optimal de l'avant-pied.

Le segment postérieur est adapté pour recevoir au moins une partie de la face inférieure du talon, et être disposé en regard et en dessous d'au moins une partie de la face inférieure du talon, de façon à soutenir au moins partiellement la face inférieure du talon et à pouvoir exercer une force de rappel de la partie postérieure du pied vers la position de repos lors d'un déplacement relatif des segments hors de la position de repos.

Dans une variante de réalisation avantageuse d'un dispositif orthopédique selon l'invention, le segment postérieur s'étend transversalement entre une extrémité latérale interne et un rebord latéral externe, de façon à s'étendre sous toute la largeur du talon.

Dans une variante de réalisation avantageuse, un dispositif orthopédique selon l'invention permet de soutenir non seulement l'avant-pied et le talon mais également la voûte plantaire du pied. Ce soutien est particulièrement important lorsque le trouble de la marche s'accompagne d'un affaissement de la voûte plantaire ou même dans les cas d'écrasement du pied. Avantageusement, un dispositif orthopédique selon l'invention comprend un appendice adapté pour recevoir la voûte plantaire et en particulier former un soutien de voûte plantaire.

Avantageusement et selon l'invention, l'arceau supérieur présente un appendice adapté pour s'étendre en regard de la voûte plantaire. Cet appendice est en particulier adapté pour s'étendre au contact de la voûte plantaire, notamment lorsque le pied est en position de repos.

Dans une variante de réalisation particulièrement avantageuse d'un dispositif orthopédique selon l'invention, l'arceau supérieur présente un appendice interne s'étendant latéralement et vers le bas, longitudinalement disposé entre les segments antérieur et postérieur, de façon à former un soutien de voûte plantaire. Ledit appendice interne de l'arceau supérieur est disposé du côté interne du pied et est adapté pour recevoir un bord médian inférieur interne du pied. Ledit appendice interne peut notamment s'étendre sous le pied, au moins sur une portion de la largeur du pied, par exemple jusqu'à la moitié de la largeur du pied ou avantageusement sous presque toute la largeur du pied de façon à assurer une surface de réception plus importante de la voûte plantaire.

Un dispositif orthopédique selon l'invention peut être adapté pour pouvoir être porté à l'intérieur d'une chaussure, notamment une chaussure fermée, sans nécessiter de dispositif de fixation complémentaire.

Dans une variante de réalisation particulièrement avantageuse d'un dispositif orthopédique selon l'invention, le segment antérieur présente un rebord latéral interne adapté pour soutenir latéralement le gros orteil du pied. Un tel rebord latéral interne du segment antérieur consiste en un prolongement vertical de ladite première plaque. Il permet de maintenir le pied dans une position anatomiquement correcte à l'intérieur de la chaussure et évite en particulier une déviation latérale de l'avant-pied dans la chaussure.

Dans une variante de réalisation d'un dispositif orthopédique selon l'invention, le segment postérieur comprend un appendice talonnier s'étendant vers le haut, adapté pour recevoir la face postérieure du talon. Ledit appendice talonnier est adapté pour s'étendre depuis le segment postérieur vers la jambe et le mollet, par exemple sous la forme d'une collerette talonnière de manière à former une cuvette adaptée pour recevoir le talon. Un tel appendice talonnier permet d'améliorer le maintien du pied au sein du dispositif orthopédique et de stabiliser le pied lors des différentes phases de la marche, notamment à l'attaque du pas, lors de l'appui sur le sol avec le talon.

Dans une autre variante de réalisation d'un dispositif orthopédique selon l'invention, celui-ci comprend une portion adaptée pour être en contact avec le cou-de-pied, ladite portion étant reliée élastiquement au segment postérieur.

Un dispositif orthopédique selon l'invention présente ainsi une partie, dite partie inférieure, adapté pour recevoir et soutenir un pied. La partie inférieure comprend au moins le segment antérieur, le segment postérieur et l'arceau supérieur. Le dispositif orthopédique selon l'invention peut être utilisé uniquement composé de cette partie inférieure.

En variante un dispositif orthopédique selon l'invention comprend une telle partie inférieure prolongée par une partie, dite partie jambière, s'étendant vers le haut à partir de la partie inférieure (vers la jambe et éventuellement jusqu'à la cuisse). Ainsi, dans une variante de réalisation particulièrement avantageuse de l'invention, le dispositif orthopédique comprend en outre un segment supérieur adapté pour recevoir au moins une partie de la jambe dudit membre inférieur humain.

Dans tout le texte, on entend par "jambe", la partie de chaque membre inférieur humain s'étendant du genou au pied.

Dans tout le texte, on entend par "loge antérieure", la partie de la jambe contenant le muscle tibial antérieur, le muscle extenseur propre du gros orteil, le muscle long extenseur des orteils et le péronier antérieur.

Dans tout le texte, on entend par "loge externe" ou "loge latérale", la partie de la jambe contenant les muscles long et court fibulaires.

Ainsi, dans tout le texte, on entend par "loge antéro-externe" ou "loge antéro-latérale", la partie de la jambe contenant les muscles de la loge antérieure et les muscles de la loge externe (ou latérale).

Dans tout le texte, on entend par "loge postérieure", la partie de la jambe contenant le muscle triceps, le muscle long fléchisseur du gros orteil, le muscle long fléchisseur des orteils et le muscle tibial postérieur.

Le segment supérieur est adapté pour s'étendre au contact d'au moins une partie de jambe portant le dispositif orthopédique. Dans une variante de réalisation particulièrement avantageuse d'un dispositif orthopédique selon l'invention, le segment supérieur est adapté pour s'étendre au moins partiellement au contact de la loge antérieure de la jambe.

Ainsi, le segment supérieur d'un dispositif orthopédique selon l'invention ne s'étend pas uniquement au contact de la loge postérieure de la jambe, tel un dispositif releveur connu de l'état de la technique, mais également au contact de la loge antérieure de la jambe.

Un dispositif orthopédique selon l'invention comprend un segment supérieur présentant au moins une portion adaptée pour former un appui antérieur en contact avec la loge antérieure de la jambe.

En particulier, un dispositif orthopédique selon l'invention comprend un segment supérieur :
- s'étendant au-dessus du segment postérieur en ménageant un logement de réception de la jambe,
- présentant :
   ∘ au moins une portion adaptée pour former un appui antérieur en contact avec la loge antérieure de la jambe, et
   ∘ au moins une portion adaptée pour former un appui postérieur en contact avec la loge postérieure de la jambe,
ledit segment supérieur étant relié élastiquement au segment postérieur de façon à :
- maintenir le segment postérieur en position de repos,
- permettre un déplacement relatif du segment supérieur et du segment postérieur l'un par rapport à l'autre, et à rappeler élastiquement le segment supérieur et le segment postérieur en position de repos.

Avantageusement et selon l'invention, le segment supérieur est relié élastiquement au segment postérieur de façon à permettre un déplacement relatif du segment supérieur et du segment postérieur l'un par rapport à l'autre dans un plan sagittal.

Le segment supérieur permet d'améliorer le maintien du membre inférieur en renforçant l'effet de maintien du pied, tout en permettant la dorsiflexion de la jambe vers le pied (autour de l'axe de la cheville). L'appui formé par le segment supérieur autour de la jambe portant le dispositif orthopédique permet d'améliorer l'effet releveur de l'avant-pied du dispositif orthopédique et surtout d'amplifier l'effet de propulsion procuré par le dispositif orthopédique en fin de pas.

Le segment supérieur s'étendant entre au moins une portion formant un appui antérieur en contact avec la loge antérieure, et notamment la loge antéro-latérale, de la jambe et au moins une portion formant un appui postérieur en contact avec la loge postérieure de la jambe, il permet également d'améliorer la stabilisation latérale du pied et de la jambe, en position statique comme au cours de la marche ou de la course à pied, en aidant au maintien de la jambe et du pied dans un même plan sagittal.

Avantageusement et selon l'invention, la partie jambière comprend un dispositif d'ouverture/fermeture s'étendant autour dudit membre inférieur humain.

Un dispositif orthopédique selon l'invention peut de la même façon également comprendre au moins une portion adaptée pour former un appui antérieur en contact avec le cou-de-pied permettant d'amplifier l'effet de propulsion procuré par le dispositif orthopédique en fin de pas.

Dans une variante de réalisation de l'invention, la partie jambière d'un dispositif orthopédique selon l'invention comprend une portion supérieure s'étendant vers le haut de façon à s'étendre au-dessus du genou dudit membre inférieur humain. Le dispositif orthopédique selon cette variante de l'invention peut ainsi former une orthèse cruro-pédieuse, utile notamment dans les cas de déficiences musculaires acquises traumatiques ou congénitales de la jambe.

Un dispositif orthopédique selon l'invention, complété ou non d'une partie jambière peut être formé de tout matériau adapté pour former la partie inférieure apte à soutenir le pied, avec le segment antérieur et le segment postérieur selon l'invention, reliés élastiquement par au moins un arceau supérieur. Un dispositif orthopédique selon l'invention peut en particulier être formé de tout matériau choisi parmi les matériaux métalliques, les matériaux polymères et les matériaux composites à matrice polymère et/ou métallique.

En particulier, un dispositif orthopédique selon l'invention est formé de tout matériau présentant une tenue en fatigue améliorée, de façon à supporter des déformations répétées subies lors de son utilisation (déformations liées aux mouvements du pied et de la jambe lors de la marche en particulier).

Dans une variante de réalisation particulièrement avantageuse d'un dispositif orthopédique selon l'invention, celui-ci est formé d'un seul tenant. En particulier, un dispositif orthopédique selon l'invention est entièrement formé en un seul bloc issu de moulage. Il est ainsi formé d'un même matériau façonné en une structure continue adaptée pour soutenir le membre inférieur et transmettre efficacement les efforts subis par le dispositif orthopédique. Ainsi, avantageusement et selon l'invention, le segment antérieur, l'arceau et le segment postérieur sont formés d'un même matériau façonné en une structure continue adaptée pour transmettre les efforts exercés lors de la marche ou de tout mouvement du pied sur les différentes parties du dispositif orthopédique.

Le choix d'un même matériau constitutif pour former à la fois le segment antérieur, l'arceau et le segment postérieur présente en outre l'avantage de simplifier le procédé et le coût de fabrication du dispositif orthopédique.

En outre, l'inventeur s'est rendu compte qu'il est possible de réaliser un tel dispositif orthopédique qui soit léger et d'un faible encombrement, tout en présentant un soutien et une résistance mécanique suffisants en formant une structure mécanique principale (définissant le comportement mécanique statique et dynamique du dispositif orthopédique) par un matériau composite polymère comprenant des renforts fibreux.

Avantageusement et selon l'invention, le dispositif orthopédique comprend une structure mécanique principale formée d'un matériau composite comprenant au moins un renfort sous forme de fibres et au moins un matériau polymère au sein duquel s'étendent les fibres. Avantageusement et selon l'invention, ladite structure mécanique principale du dispositif orthopédique est formée d'un matériau composite comprenant des fibres tissées ou non tissées et au moins un matériau polymère au sein duquel s'étendent les fibres. Chaque renfort sous forme de fibres est par exemple choisi parmi les fibres de carbone, les fibres aramides et les fibres de verre. Le matériau polymère au sein duquel s'étendent les fibres est choisi parmi les matériaux polymères thermodurcissables et les matériaux polymères thermoplastiques adaptés pour préparer un dispositif orthopédique selon l'invention. Le matériau polymère est par exemple choisi parmi les résines époxy ou encore les résines vinylesters.

Avantageusement et selon l'invention, ladite structure mécanique principale est une bande mince préformée - notamment comprenant au moins une portion hélicoïdale- en matériau composite, rigide, élastique en flexion. Dans les cas où le dispositif orthopédique est destiné à être disposé à l'intérieur d'une chaussure ou utilisé seul, cette bande rigide élastique est avantageusement complétée du côté interne destiné à venir au contact du patient par au moins une couche de matériau de rembourrage, notamment une couche mince de mousse polymère choisie dans le groupe formé des mousses polyuréthanes, des mousses polyéthers et des mousses polyéthylènes.

Un dispositif orthopédique selon l'invention ayant une structure mécanique principale formée en matériau composite à matrice polymère présente l'avantage d'être mince et est donc adapté pour pouvoir être porté dans une chaussure, notamment dans des chaussures de sport ou de ville.

Un dispositif orthopédique selon l'invention présentant en outre une partie jambière est adapté pour permettre tous les mouvements relatifs du pied, de la cheville et de la jambe, normalement associés à la station debout, à la marche et à la course, c'est-à-dire les mouvements normalement permis par l'articulation de la cheville. Avantageusement et selon l'invention, la partie pédieuse et la partie jambière sont adaptées pour former entre elles un angle compris entre 60° et 120°.

L'invention s'étend à une chaussure équipée d'un dispositif orthopédique selon l'invention. L'invention concerne donc également une chaussure équipée d'un dispositif orthopédique pour un membre inférieur humain comprenant :
- un segment antérieur adapté pour recevoir la plante d'un avant-pied d'un pied,
- un segment postérieur adapté pour recevoir la face inférieure du talon dudit pied, dans lequel :
   - le segment antérieur et le segment postérieur sont formés de deux pièces distinctes l'une de l'autre,
   - le segment antérieur et le segment postérieur sont reliés par au moins un arceau supérieur :
      ∘ s'étendant au-dessus du segment antérieur et du segment postérieur en ménageant un logement de réception du pied,
      ∘ adapté pour maintenir le segment antérieur et le segment postérieur dans une position de repos, caractérisée en ce que :
         l'arceau supérieur est élastique et adapté pour permettre un déplacement relatif du segment antérieur et du segment postérieur l'un par rapport à l'autre, à partir de la position de repos, et pour rappeler élastiquement le segment antérieur et le segment postérieur en position de repos.

Le dispositif orthopédique équipant une telle chaussure peut également présenter un segment supérieur tel que défini précédemment. Dans une variante de réalisation avantageuse, un tel segment supérieur présente une portion adaptée pour former un appui antérieur en contact avec le cou-de-pied.

Un dispositif orthopédique selon l'invention peut équiper tout type de chaussure, en particulier tout type de chaussure fermée, par exemple une chaussure montante adaptée pour maintenir la cheville. Le dispositif orthopédique est inséré dans la chaussure de façon à être disposé entre le pied et le logement interne de la chaussure. Une chaussure équipée d'un dispositif orthopédique selon l'invention permet donc d'optimiser l'efficacité d'une orthèse pédieuse selon l'invention en améliorant le maintien du pied, tout en étant plus esthétique et plus pratique pour protéger le pied de l'environnement extérieur.

L'invention s'étend à une prothèse d'au moins une portion d'un membre inférieur humain équipée d'un dispositif orthopédique selon l'invention. L'invention concerne donc également une prothèse d'au moins une portion d'un membre inférieur humain, ladite prothèse étant équipée d'un dispositif orthopédique comprenant :
- un segment antérieur adapté pour recevoir la plante d'un avant-pied d'un pied,
- un segment postérieur adapté pour recevoir la face inférieure du talon dudit pied, dans lequel :
   - le segment antérieur et le segment postérieur sont formés de deux pièces distinctes l'une de l'autre,
   - le segment antérieur et le segment postérieur sont reliés par au moins un arceau supérieur :
      ∘ s'étendant au-dessus du segment antérieur et du segment postérieur en ménageant un logement de réception du pied,
      ∘ adapté pour maintenir le segment antérieur et le segment postérieur dans une position de repos, caractérisée en ce que :
         l'arceau supérieur est élastique et adapté pour permettre un déplacement relatif du segment antérieur et du segment postérieur l'un par rapport à l'autre, à partir de la position de repos, et pour rappeler élastiquement le segment antérieur et le segment postérieur en position de repos.

Avantageusement et selon l'invention, ladite prothèse est équipée d'un dispositif orthopédique selon l'invention comprenant un segment supérieur s'étendant au-dessus du segment postérieur en ménageant un logement de réception de la jambe,
- présentant :
   ∘ au moins une portion adaptée pour former un appui antérieur en contact avec la loge antérieure de la jambe, et
   ∘ au moins une portion adaptée pour former un appui postérieur en contact avec la loge postérieure de la jambe,
ledit segment supérieur étant relié élastiquement au segment postérieur de façon à :
- maintenir le segment postérieur en position de repos,
- permettre un déplacement relatif du segment supérieur et du segment postérieur l'un par rapport à l'autre, et à rappeler élastiquement le segment supérieur et le segment postérieur en position de repos.

Avantageusement et selon l'invention, la prothèse d'au moins une portion d'un membre inférieur humain équipée d'un dispositif orthopédique selon l'invention est choisie parmi les prothèses de membre inférieur humain (prothèses du pied ou prothèses du pied et de la jambe notamment) adaptées pour permettre des mouvements de flexion plantaire et, des mouvements de dorsiflexion autour de l'axe de la cheville (c'est-à-dire autour de l'axe bi-malléolaire). Une telle prothèse présente également, de préférence, une résistance mécanique adaptée pour permettre un appui au moins en partie vertical du poids du corps de la personne portant la prothèse. La mobilité d'une telle prothèse est donc très semblable à celle d'un membre inférieur humain, en autorisant tout mouvement normalement permis par un membre inférieur, notamment tout mouvement associés à la marche ou à la course à pied, et tout en présentant une résistance imitant une jambe et un pied humains vivants. Il n'est pas indispensable d'utiliser pour ce faire une prothèse commandée électroniquement, une prothèse autorisant les mouvements normalement permis par un membre inférieur humain étant suffisante.

Un dispositif orthopédique selon l'invention peut être fabriqué par tout procédé de mise en oeuvre et de préparation de matériaux composites à matrice polymère. En particulier, un dispositif orthopédique selon l'invention peut être fabriqué par tout procédé de moulage, par exemple par moulage au contact sur une empreinte ou par moulage à l'aide de tissus pré-imprégnés d'une matrice polymère. Le procédé de fabrication d'un dispositif orthopédique selon l'invention peut comprendre une étape de polymérisation sous vide et/ou sous pression. Un dispositif orthopédique selon l'invention peut également être fabriqué par un procédé d'infusion ou d'injection de résine polymère liquide. Pour ce faire, il est possible d'utiliser un moule représentatif d'un pied ou d'un membre inférieur humain.

D'autres buts, caractéristiques et avantages de l'invention apparaissent à la lecture de la description suivante d'un de ses modes de réalisation préférentielle donnée à titre d'exemple non limitatif, et qui se réfère aux figures annexées dans lesquelles :
- la figure 1 est une vue schématique en perspective d'un premier mode de réalisation d'un dispositif orthopédique selon l'invention,
- la figure 2 est une vue schématique de dessous d'un premier mode de réalisation d'un dispositif orthopédique selon l'invention,
- la figure 3 est une vue schématique en perspective d'un deuxième mode de réalisation d'un dispositif orthopédique selon l'invention comprenant en outre une partie jambière,
- la figure 4 est une vue schématique en perspective d'un troisième mode de réalisation d'un dispositif orthopédique selon l'invention,
- la figure 5 est une vue schématique en perspective d'un quatrième mode de réalisation d'un dispositif orthopédique selon l'invention comprenant une partie jambière.

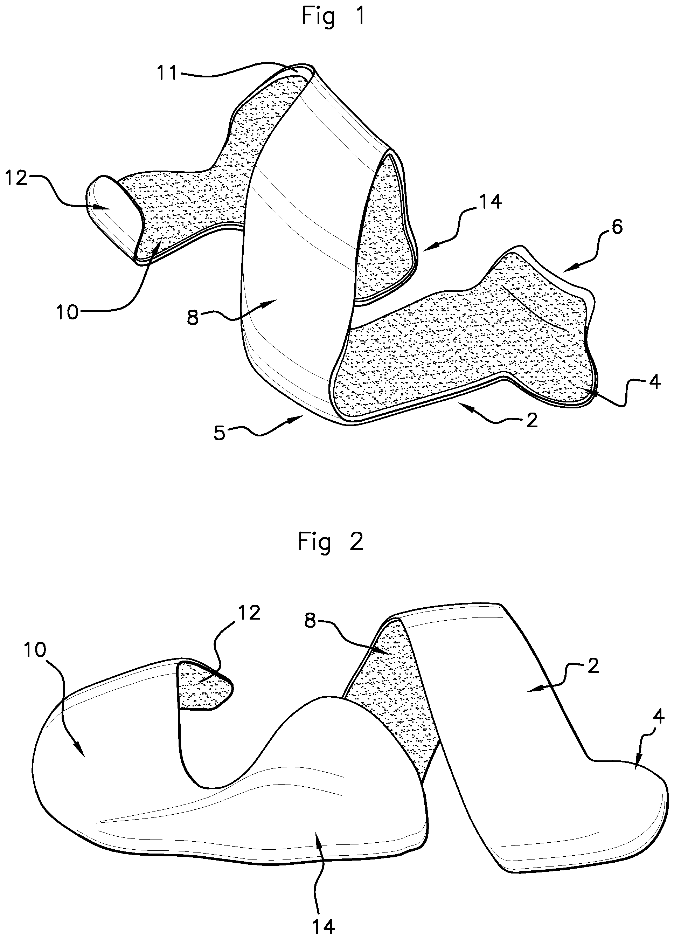

Selon un premier mode de réalisation selon l'invention représenté aux figures 1 et 2, le dispositif orthopédique est une orthèse pédieuse se présentant sous la forme d'un ruban enroulé, globalement hélicoïdal, comprenant une couche externe d'un matériau rigide, ménageant un logement pour un pied, de façon à ce que le ruban semble enroulé autour du pied depuis l'avant-pied jusqu'au talon.

L'orthèse pédieuse selon l'invention représentée aux figures 1 et 2 est adaptée pour recevoir un pied droit humain.

La couche externe de l'orthèse pédieuse représentée aux figures 1 et 2 est formée d'une résine époxy au sein de laquelle s'étendent, à titre de renfort, des fibres de carbone, par exemple sous forme de deux ou trois couches d'un tissu sergé ou taffetas, de préférence non unidirectionnel.

Le ruban formant l'orthèse présente une épaisseur telle qu'il est possible de la porter à l'intérieur d'une chaussure. L'épaisseur du matériau formant l'orthèse (hors épaisseur de la couche de matériau de rembourrage éventuelle) est par exemple comprise entre 0,2 mm et 5 mm, et notamment entre 0,5 mm et 2 mm. Le ruban formant l'orthèse présente par exemple une largeur moyenne comprise entre 1 cm et 7 cm, et notamment entre 1,5 cm et 4 cm.

A l'avant du pied, du côté interne du pied (où se situe le gros orteil) l'orthèse est adaptée pour s'étendre entre un rebord 6 latéral interne adapté pour soutenir latéralement le gros orteil du pied et un bord 5 latéral externe adapté pour recevoir le cinquième métatarse du pied en formant un segment 2 antérieur.

Le segment 2 antérieur s'étend donc sous la forme d'une première plaque, entre un bord latéral adapté pour recevoir le gros orteil et le bord 5 latéral externe adapté pour recevoir le cinquième métatarse du pied. Le segment antérieur est adapté pour s'étendre sous toute la largeur de l'avant-pied et pour exercer un soutien optimal de l'avant-pied, en l'occurrence un soutien de chacun des cinq métatarses.

Le segment 2 antérieur présente un appendice 4 avant adapté pour recevoir le gros orteil. L'appendice 4 avant est adapté pour être disposé en regard de la face inférieure du gros orteil du pied. Le gros orteil (ou hallux) repose au moins partiellement sur l'appendice 4 avant du segment 2 antérieur lorsque la personne portant l'orthèse se tient debout en appui sur son pied ou lors des phases d'appui de la marche ou de la course à pied.

Du côté externe à l'avant du pied, où se situe le petit orteil (ou quintus), le bord 5 latéral externe du segment 2 antérieur de l'orthèse se prolonge vers le haut, au-dessus du pied, sous la forme d'un arceau 8 supérieur rigide mais élastique en flexion. L'arceau 8 supérieur est formé d'une portion dudit ruban enroulé sur lui-même et qui s'étend jusqu'à la région malléolaire interne du pied et se prolonge sous le talon en formant un segment 10 postérieur. L'arceau 8 supérieur s'étend obliquement au contact de la face supérieure du pied, entre une extrémité latérale externe du segment 2 antérieur et une extrémité latérale interne du segment 10 postérieur. L'arceau 8 supérieur forme ainsi un logement dans lequel un pied peut être introduit, au moins dans la position de repos, à la façon d'une chaussure.

Le segment 10 postérieur s'étend sous la forme d'une deuxième plaque, entre une extrémité latérale interne et un rebord 12 latéral externe, de façon à s'étendre sous toute la largeur du talon et à soutenir celui-ci.

L'arceau 8 supérieur présente un appendice 14 interne adapté pour soutenir au moins partiellement la voûte plantaire. L'appendice 14 interne de l'arceau 8 supérieur est disposé du côté interne du pied s'étend latéralement et sous le pied en formant une arche interne adaptée pour suivre la courbure de la voûte plantaire.

Le segment 2 antérieur et le segment 10 postérieur constituent ainsi deux appuis pour le pied, respectivement pour l'avant-pied et le talon. Ces deux segments sont reliés par l'arceau 8 supérieur rigide élastique en flexion adapté d'une part, pour maintenir le segment 2 antérieur et le segment 10 postérieur dans une position de repos, et, d'autre part, pour permettre un déplacement relatif du segment 2 antérieur et du segment 10 postérieur l'un par rapport à l'autre, à partir de la position de repos, et pour les rappeler élastiquement en position de repos. La position de repos de l'orthèse selon l'invention est sensiblement identique seule ou avec un pied introduit à l'intérieur. La position de repos de l'orthèse correspond en particulier à une position normale du pied et de la jambe, lors de la station debout par exemple.

L'orthèse pédieuse selon l'invention est adaptée pour être au contact du pied ou éventuellement s'ajuster d'elle-même de façon à venir en contact du pied, notamment en position de repos. Une telle orthèse est facile à mettre en enfilant simplement le pied au sein du logement de réception du pied ménagé par le segment 2 antérieur, le segment 10 postérieur et l'arceau 8 supérieur.

L'arceau 8 supérieur permet au segment 2 antérieur et au segment 10 postérieur de se déplacer l'un par rapport à l'autre, à la fois selon la direction longitudinale du pied et selon une direction perpendiculaire à la direction longitudinale du pied. L'orthèse est ainsi adaptée pour accompagner tous les déplacements de l'avant-pied et du talon, notamment lors de la marche ou de la course à pied.

L'arceau 8 supérieur est non seulement adapté pour permettre un déplacement relatif du segment 2 antérieur et du segment 10 postérieur l'un par rapport à l'autre, à partir de la position de repos, mais également pour rappeler élastiquement le segment 2 antérieur et le segment 10 postérieur en position de repos.

L'arceau 8 supérieur reliant le segment 2 antérieur et le segment 10 postérieur agissent ainsi à la façon d'un ressort ou d'un arc restituant de l'énergie potentielle emmagasinée lors de la déformation élastique de l'arceau 8 supérieur quand le segment 2 antérieur et le segment 10 postérieur sont déplacés de la position de repos, sous forme d'énergie cinétique permettant un effet releveur dynamique de l'avant-pied et un effet de propulsion du talon en fin de pas.

L'orthèse permet donc de faciliter la marche en maintenant le pied dans une position anatomiquement correcte, mais également en procurant un effet de propulsion en fin de pas. Lors de la marche, l'effet de propulsion se manifeste en particulier à la fin de la phase d'appui, lorsque le talon se soulève et que seuls l'avant-pied et les orteils restent encore en contact avec le sol. Le segment 2 antérieur et le segment 10 postérieur sont alors déplacés l'un par rapport à l'autre, hors de la position de repos et l'arceau 8 supérieur est mis en tension. L'énergie potentielle élastique emmagasinée par l'orthèse lors de la flexion de la jambe vers l'avant qui accompagne cette étape de la marche est ensuite restituée en produisant, en fin de pas, un effet propulseur du pied vers l'avant, lorsque les orteils quittent leur appui au sol.

En outre, l'orthèse permet également de soutenir efficacement la voûte plantaire, notamment lors de la flexion de la jambe vers l'avant et que le talon se soulève. En effet, la mise en tension de l'orthèse entraîne également une mise en tension de l'appendice 14 interne formant un soutien de la voûte plantaire et permet de maintenir d'autant plus la voûte plantaire et d'éviter son affaissement.

Par ailleurs, pour un plus grand confort un film 11 mince en matériau alvéolaire telle qu'une mousse synthétique est disposée et collée sur la surface interne de l'orthèse, c'est-à-dire la surface destinée à être en contact avec le pied.

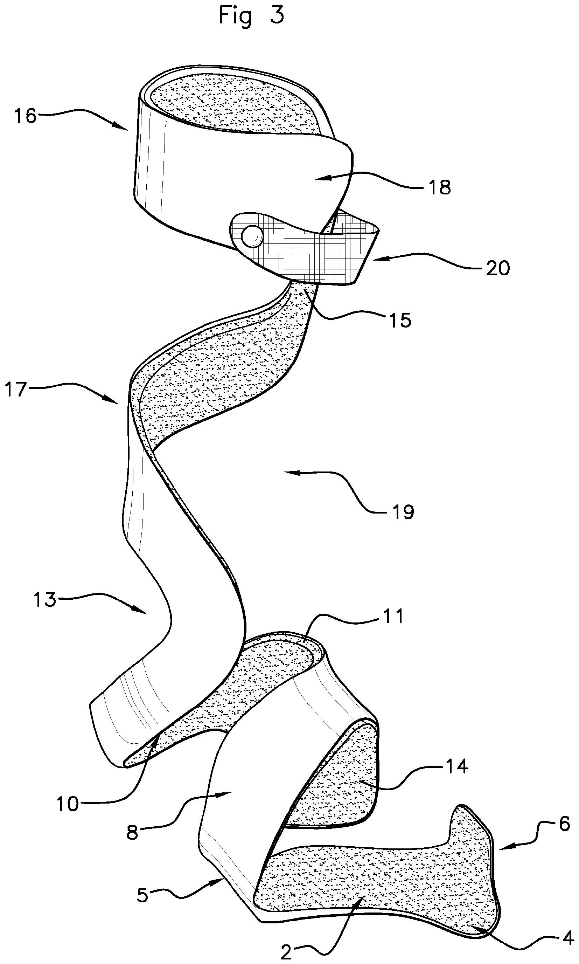

Selon un deuxième mode de réalisation d'un dispositif orthopédique selon l'invention représenté en figure 3, celui est une orthèse pédieuse se présentant sous la forme d'un ruban hélicoïdal formé d'un matériau rigide, ménageant un logement pour un membre inférieur droit humain, de façon à ce que le ruban semble enroulé autour du pied depuis l'avant-pied jusqu'au talon puis autour du mollet, jusque sous le genou.

L'orthèse pédieuse selon l'invention représentée en figure 3 présentant une partie inférieure, ménageant un logement pour le pied, et une partie supérieure, ménageant un logement pour la jambe. La partie inférieure de ce deuxième mode de réalisation de l'orthèse est identique à l'orthèse selon le premier mode de réalisation décrite précédemment et représentée aux figures 1 et 2. Cependant, le rebord latéral externe du segment 10 postérieur n'est pas interrompu au niveau de la malléole externe mais se prolonge en formant la partie supérieure de l'orthèse.

La partie supérieure de l'orthèse est formée d'un segment supérieur 19 adapté pour s'étendre au contact d'au moins une partie de la jambe portant l'orthèse, et en particulier pour s'étendre au moins partiellement au contact de la loge antéro-externe de la jambe formant ainsi un appui de la face avant de la jambe.

Le segment supérieur 19 comprend une première partie 13 courbe s'étendant depuis le segment 10 postérieur de la partie inférieure de l'orthèse vers le haut de façon à former un appui au contact de la loge antéro-externe de la jambe. La première partie 13 prend la forme d'un arc de cercle s'étendant de la malléole externe à mi-hauteur du mollet. Elle se prolonge en une deuxième partie 17 du segment supérieur 19 s'étendant à l'arrière et autour du mollet. La deuxième partie 17 s'étend ensuite vers le haut en formant une troisième partie 15 du segment supérieur 19. La troisième partie 15 forme un arc de cercle s'étendant sur le côté interne du mollet vers le haut. Sous le genou, la troisième partie 15 se prolonge en une quatrième partie 16 s'étendant à l'arrière et autour du haut du mollet jusqu'à une cinquième partie 18, en formant un anneau ouvert à l'avant de la jambe. La cinquième partie 18 présente un bord terminal supérieur auquel est fixée une attache avec un ruban souple auto-agrippant 20. Une boucle est fixée au niveau de la troisième partie 15 à travers laquelle le ruban souple auto-agrippant 20 peut être passé de façon à former un dispositif d'ouverture/fermeture.

Le segment supérieur 19 permet d'améliorer le maintien du membre inférieur en renforçant l'effet de maintien du pied. L'appui formé par le segment supérieur 19 autour de la jambe portant l'orthèse permet d'améliorer l'effet releveur de l'avant-pied de l'orthèse et d'amplifier l'effet de propulsion en fin de pas procuré par l'orthèse pédieuse. Le segment supérieur 19 permet également d'améliorer l'effet de stabilisation latérale du pied et de la jambe, en aidant au maintien de la jambe et du pied dans un même plan sagittal, sans toutefois empêcher les mouvements latéraux de la jambe par rapport au pied.

Une orthèse selon ce deuxième mode de réalisation permet non seulement de faciliter la marche en maintenant le pied et la jambe dans une position anatomiquement correcte, mais également en procurant un effet de propulsion en fin de pas. L'effet de propulsion est encore amplifié par rapport au premier mode de réalisation d'une orthèse selon l'invention (c'est-à-dire avec une partie supérieure) la première partie 13 et la cinquième partie 18 dudit segment supérieur 19 formant des appuis antéro-externes et antérieurs et la deuxième partie 17 et la quatrième partie 16 dudit segment supérieur 19 formant des appuis postérieurs, l'ensemble permettant également de mettre en tension l'orthèse et de procurer un effet de propulsion amélioré de la jambe et du pied en fin de pas.

Une orthèse pédieuse telle qu'une orthèse selon ce deuxième mode de réalisation est facile à mettre, par exemple en enfilant d'abord le pied au sein du logement de réception du pied ménagé par le segment 2 antérieur, le segment 10 postérieur et l'arceau 8 supérieur, tout en maintenant d'une main la partie supérieure vers l'arrière ou l'extérieur, puis en ramenant la partie supérieure autour de la jambe.

Une mousse synthétique sous la forme d'un film 11 mince en matériau alvéolaire est collée sur la surface interne de l'orthèse destinée à être en contact avec le pied et la jambe, dans le cas où l'orthèse est utilisée à l'intérieur d'une chaussure. La mousse synthétique est avantageusement choisie parmi les mousses synthétiques adaptées pour être contact avec la peau humaine (mousses non allergisantes en particulier).

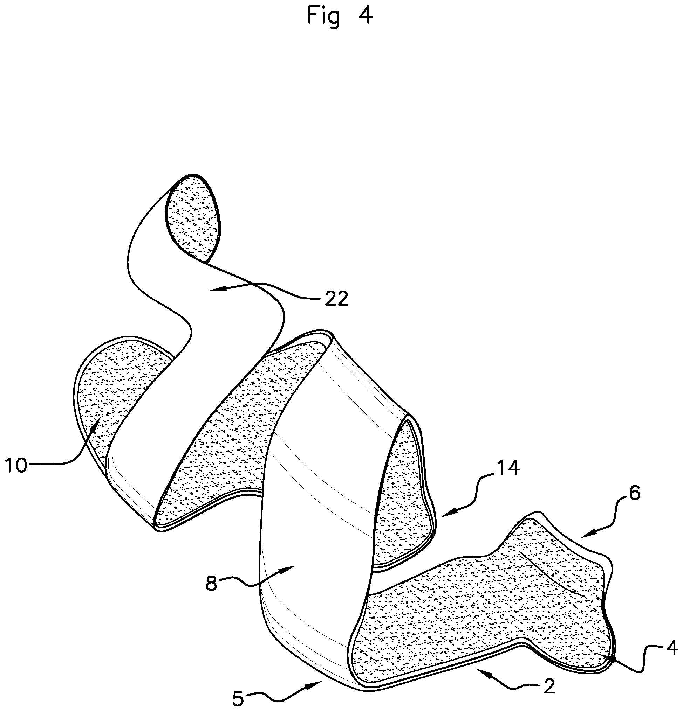

Selon un troisième mode de réalisation d'un dispositif orthopédique selon l'invention représenté en figure 4, celui-ci est une orthèse pédieuse se présentant sous la forme d'un ruban hélicoïdal formé d'un matériau rigide, ménageant un logement pour un pied, telle une orthèse selon le premier mode de réalisation représenté en figures 1 et 2, mais prolongée depuis le rebord 12 latéral externe vers l'avant de la cheville par une portion 22 adaptée pour être en contact avec le cou-de-pied. Le ruban parait donc être enroulé autour du pied depuis l'avant-pied jusqu'au talon puis sur le dessus du pied au niveau du cou-de-pied.

L'arceau 8 supérieur présente un appendice 14 interne adapté pour soutenir au moins partiellement la voûte plantaire. Dans un mode de réalisation particulièrement avantageux, l'appendice interne peut également se présenter sous la forme d'un tissu élastique adapté pour s'étendre sous la voûte plantaire et former un soutien de voûte plantaire.

Une orthèse selon le troisième mode de réalisation peut par exemple être utilisée seule ou dans une chaussure montante, telle qu'une chaussure de marche ou de randonnée.

Dans le cas où l'orthèse équipe une chaussure, l'orthèse peut être disposée dans la chaussure de façon à être amovible ou bien y être fixée, par exemple par une couture entre un bord du tissu élastique formant l'appendice interne soutien de voûte plantaire.

De cette façon, le port d'une telle orthèse permet de faciliter la marche à la fois en maintenant le pied dans une position anatomiquement correcte et en procurant un effet de propulsion en fin de pas. Cet effet de propulsion en fin de pas est notamment amélioré grâce à l'appui antérieur formé par la portion 22 de l'orthèse au niveau du cou-de-pied. Ainsi, lors de la marche, à la fin de la phase d'appui, lorsque le talon se soulève et que seuls l'avant-pied et les orteils restent encore en contact avec le sol, le segment antérieur et le segment postérieur sont déplacés l'un par rapport à l'autre, hors de la position de repos, l'arceau supérieur est mis en tension. La portion 22 de l'orthèse au niveau du cou-de-pied est également déplacée vers l'avant par appui de la cheville et contribue à mettre en tension l'orthèse. L'énergie potentielle emmagasinée par l'orthèse lors de cette flexion de la jambe vers l'avant est alors ensuite restituée en produisant, en fin de pas, un effet propulseur du pied vers l'avant, lorsque les orteils quittent leur appui au sol.

La portion 22 de l'orthèse peut également être prolongée vers l'arrière de la cheville de façon à mieux maintenir le pied.

En outre, lors de la flexion de la jambe vers l'avant (talon soulevé), la mise en tension de l'orthèse entraîne également une mise en tension de l'appendice interne formant un soutien de la voûte plantaire et permet de maintenir d'autant plus la voûte plantaire et d'éviter son affaissement en particulier lors de cette phase de la marche (jambe fléchie, talon soulevé et avant-pied en appui au sol).

Une orthèse selon ce troisième mode de réalisation d'un dispositif orthopédique selon l'invention peut avantageusement être portée ou être intégrée dans une chaussure montante au-dessus de la cheville, telle une chaussure de sport ou de randonnée ou toute autre chaussure orthopédique. Elle procure un effet de propulsion amélioré par rapport à une orthèse selon le premier mode de réalisation grâce à l'appui antérieur au niveau du cou-de-pied formé par la portion 22 de l'orthèse. Une telle orthèse permet ainsi de faciliter la marche et de rendre plus aisés les efforts à fournir lors d'une activité physique telle que la course à pied ou la randonnée.

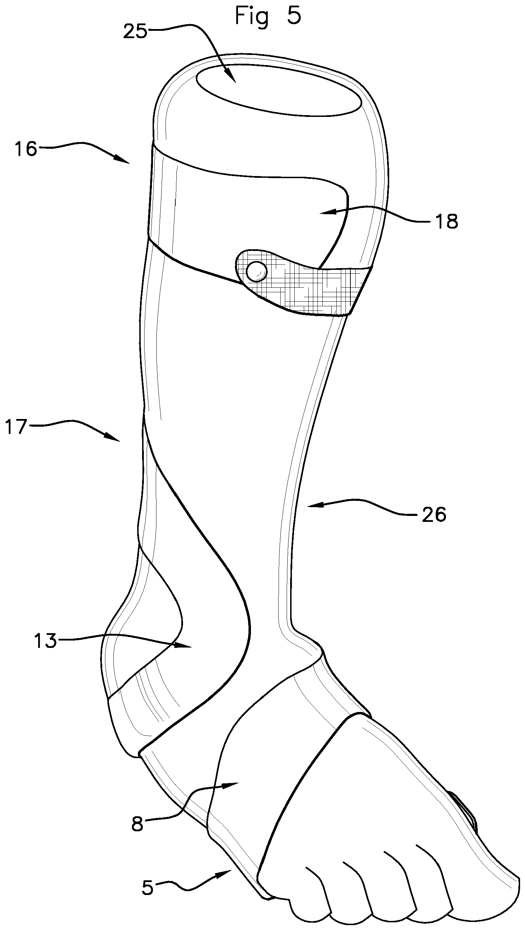

Selon un quatrième mode de réalisation d'un dispositif orthopédique selon l'invention représenté en figure 5, le dispositif orthopédique se présente sous la forme d'une prothèse. Dans ce mode de réalisation, une orthèse selon le deuxième mode de réalisation de l'invention telle que représentée en figure 3 est associée à une prothèse 26 d'un membre inférieur. Une orthèse telle que représentée en figure 3 est ainsi intégrée à titre de lame dans une prothèse de membre inférieur par moulage simultané ou collage ou simplement disposée autour ou à l'intérieur de la prothèse. L'aspect et la mobilité de la prothèse 26 (notamment la mobilité de l'articulation de la cheville) sont très semblables à ceux d'un membre inférieur humain. La prothèse 26 présente également une résistance imitant une jambe et un pied vivants. La prothèse 26 de membre inférieur comporte une extrémité supérieure 25 pouvant être fixée à un moignon tibial. En particulier, la prothèse 26 permet les mouvements de flexion plantaire et de dorsiflexion autour de l'axe bi-malléolaire, tout en présentant une résistance semblable à celle d'une jambe et d'un pied vivants.

Une prothèse selon ce quatrième mode de réalisation permet donc, outre de remplacer un membre inférieur humain, de procurer une stabilisation et un maintien du membre inférieur ainsi qu'un effet releveur de l'avant-pied et un effet de propulsion en fin de pas, tout en laissant libre les amplitudes physiologiques des différentes articulations du membre inférieur.

Dans ce quatrième mode de réalisation d'un dispositif orthopédique selon l'invention, le film 11 mince de mousse synthétique n'est pas nécessaire. En outre, l'orthèse selon le deuxième mode de réalisation de l'invention étant par exemple intégrée à la prothèse 26, il n'est pas forcément utile de prévoir un dispositif 20 d'ouverture/fermeture par ruban auto-adhésif.

Les dispositifs orthopédiques selon l'invention, représentés en figures 1, 2, 3, 4 et 5 sont adaptés pour recevoir un pied droit et/ou une jambe droite. Bien sûr, il est possible de réaliser tout dispositif orthopédique selon l'invention pour un pied droit comme pour un pied gauche (par symétrie).

L'invention peut faire l'objet de très nombreuses variantes de réalisation. En particulier, l'orthèse peut être utilisée à l'extérieur d'une chaussure (sans nécessiter dans ce cas une couche de matériau de rembourrage interne), ou encore seule (ni à l'intérieur, ni à l'extérieur d'une chaussure). En outre, selon les cas, une même personne peut porter un dispositif orthopédique selon l'invention à un seul pied, ou un dispositif orthopédique selon l'invention à chaque pied.

## Revendications

1. Dispositif orthopédique pour un membre inférieur humain comprenant :
- un segment (2) antérieur adapté pour recevoir la plante d'un avant-pied d'un pied,
- un segment (10) postérieur adapté pour recevoir la face inférieure du talon dudit pied, dans lequel :
- le segment antérieur (2) et le segment (10) postérieur sont formés de deux pièces distinctes l'une de l'autre,
- le segment (2) antérieur et le segment (10) postérieur sont reliés par au moins un arceau (8) supérieur :
∘ s'étendant au-dessus du segment (2) antérieur et du segment (10) postérieur en ménageant un logement de réception du pied,
∘ adapté pour maintenir le segment (2) antérieur et le segment (10) postérieur dans une position de repos, **caractérisé en ce que** :
l'arceau supérieur est élastique et adapté pour permettre un déplacement relatif du segment (2) antérieur et du segment (10) postérieur l'un par rapport à l'autre, à partir de la position de repos, et pour rappeler élastiquement le segment antérieur et le segment postérieur en position de repos.

2. Dispositif selon la revendication 1, **caractérisé en ce que** le segment (2) antérieur est formé d'une première plaque et le segment (10) postérieur est formé d'une deuxième plaque, distincte de la première plaque.

3. Dispositif selon l'une des revendications 1 ou 2, **caractérisé en ce que** le segment (2) antérieur et le segment (10) postérieur s'étendent dans le prolongement longitudinal l'un de l'autre et sont longitudinalement écartés l'un de l'autre, au moins en position de repos.

4. Dispositif selon la revendication 3, **caractérisé en ce que** l'arceau supérieur est adapté pour permettre un déplacement relatif du segment (2) antérieur et du segment (10) postérieur l'un par rapport à l'autre, à partir de la position de repos, dans un plan sagittal,

5. Dispositif selon l'une des revendications 1 à 4, **caractérisé en ce que** le segment (2) antérieur est adapté pour recevoir le gros orteil du pied.

6. Dispositif selon l'une des revendications 1 à 5, **caractérisé en ce que** l'arceau (8) supérieur s'étend entre une extrémité latérale externe du segment (2) antérieur et une extrémité latérale interne du segment (10) postérieur.

7. Dispositif selon l'une des revendications 1 à 6, **caractérisé en ce que** le segment (2) antérieur s'étend transversalement entre un bord latéral interne adapté pour recevoir le gros orteil et un bord latéral externe adapté pour recevoir le cinquième métatarse du pied.

8. Dispositif selon l'une des revendications 1 à 7, **caractérisé en ce que** le segment (10) postérieur s'étend transversalement entre une extrémité latérale interne et un rebord (12) latéral externe.

9. Dispositif selon l'une des revendications 1 à 8, **caractérisé en ce que** l'arceau (8) supérieur présente un appendice (14) interne s'étendant latéralement et vers le bas, longitudinalement disposé entre les segments antérieur et postérieur, de façon à former un soutien de voûte plantaire.

10. Dispositif selon l'une des revendications 1 à 9, **caractérisé en ce que** le segment (2) antérieur présente un rebord (6) latéral interne adapté pour soutenir latéralement le gros orteil du pied.

11. Dispositif selon l'une des revendications 1 à 10, **caractérisé en ce que** le segment (10) postérieur comprend un appendice talonnier s'étendant vers le haut, adapté pour recevoir la face postérieure du talon.

12. Dispositif selon l'une des revendications 1 à 11, **caractérisé en ce qu'**il comprend un segment supérieur (19) :
- s'étendant au-dessus du segment (10) postérieur en ménageant un logement de réception de la jambe,
- présentant :
∘ au moins une portion adaptée pour former un appui antérieur en contact avec la loge antérieure de la jambe, et
∘ au moins une portion adaptée pour former un appui postérieur en contact avec la loge postérieure de la jambe,
ledit segment supérieur (19) étant relié élastiquement au segment (10) postérieur de façon à :
- maintenir le segment (10) postérieur en position de repos,
- permettre un déplacement relatif du segment supérieur (19) et du segment postérieur l'un par rapport à l'autre, et à rappeler élastiquement le segment supérieur (19) et le segment postérieur (10) en position de repos.

13. Dispositif selon l'une des revendications 1 à 12, **caractérisé en ce qu'**il est formé d'au moins un matériau composite comprenant au moins un renfort sous forme de fibres et au moins un matériau polymère au sein duquel s'étendent les fibres.

14. Chaussure **caractérisée en ce qu'**elle est équipée d'un dispositif orthopédique selon l'une des revendications précédentes.

15. Prothèse d'au moins une portion d'un membre inférieur humain, **caractérisée en ce qu'**elle est équipée d'un dispositif orthopédique selon l'une des revendications 1 à 13.

## Patentansprüche

1. Orthopädische Vorrichtung für eine menschliche untere Gliedmaße, umfassend:
- ein vorderes Segment (2), das ausgelegt ist, um die Vorderfußsohle eine Fußes aufzunehmen,
- ein hinteres Segment (10), das ausgelegt ist, um die untere Seite der Ferse des Fußes aufzunehmen, wobei:
- das vordere Segment (2) und das hintere Segment (10) aus zwei untereinander verschiedenen Teilen gebildet sind,
- das vordere Segment (2) und das hintere Segment (10) durch mindestens einen oberen kleinen Bogen (8) verbunden sind:
∘ der sich über dem vorderen Segment (2) und dem hinteren Segment (10) erstreckt und ein Lager zur Aufnahme des Fußes bildet,
∘ ausgelegt, um das vordere Segment (2) und das hintere Segment (10) in einer Ruheposition zu halten, **dadurch gekennzeichnet, dass**:
der obere kleine Bogen elastisch und ausgelegt ist, um eine relative Verschiebung des vorderen Segments (2) und des hinteren Segments (10) mit Bezug aufeinander aus der Ruheposition zu ermöglichen, und um das vordere Segment und das hintere Segment elastisch in die Ruheposition rückzusetzen.

2. Vorrichtung nach Anspruch 1, **dadurch gekennzeichnet, dass** das vordere Segment (2) aus einer ersten Platte gebildet ist und das hintere Segment (10) aus einer zweiten Platte gebildet ist, die verschieden von der ersten Platte ist.

3. Vorrichtung nach einem der Ansprüche 1 oder 2, **dadurch gekennzeichnet, dass** sich das vordere Segment (2) und das hintere Segment (10) in der längs gerichteten Verlängerung voneinander erstrecken und längs gerichtet voneinander beabstandet sind, mindestens in der Ruheposition.

4. Vorrichtung nach Anspruch 3, **dadurch gekennzeichnet, dass** der obere kleine Bogen ausgelegt ist, um eine relative Verschiebung des vorderen Segments (2) und des hinteren Segments (10) mit Bezug aufeinander aus der Ruheposition auf einer Saggitalebene zu ermöglichen.

5. Vorrichtung nach einem der Ansprüche 1 bis 4, **dadurch gekennzeichnet, dass** das vordere Segment (2) ausgelegt ist, um die große Zehe des Fußes aufzunehmen.

6. Vorrichtung nach einem der Ansprüche 1 bis 5, **dadurch gekennzeichnet, dass** sich der kleine obere Bogen (8) zwischen einem äußeren seitlichen Ende des vorderen Segments (2) und einem inneren seitlichen Ende des hinteren Segments (10) erstreckt.

7. Vorrichtung nach einem der Ansprüche 1 bis 6, **dadurch gekennzeichnet, dass** sich das vordere Segment (2) quer gerichtet zwischen einem inneren seitlichen Rand, der ausgelegt ist, um die große Zehe aufzunehmen, und einem äußeren seitlichen Rand erstreckt, der ausgelegt ist, um die fünfte Metastase des Fußes aufzunehmen.

8. Vorrichtung nach einem der Ansprüche 1 bis 7, **dadurch gekennzeichnet, dass** sich das hintere Segment (10) quer gerichtet zwischen einem inneren seitlichen Ende und einem äußeren seitlichen überstehenden Rand (12) erstreckt.

9. Vorrichtung nach einem der Ansprüche 1 bis 8, **dadurch gekennzeichnet, dass** der obere kleine Bogen (8) einen inneren Fortsatz (14) aufweist, der sich seitlich und nach unten erstreckt, längs gerichtet angeordnet dem vorderen und dem hinteren Segment angeordnet ist, um eine Stütze des Fußgewölbes zu bilden.

10. Vorrichtung nach einem der Ansprüche 1 bis 9, **dadurch gekennzeichnet, dass** das vordere Segment (2) einen inneren seitlichen überstehenden Rand (6) aufweist, der ausgelegt ist, um die große Zehe des Fußes seitlich zu stützen.

11. Vorrichtung nach einem der Ansprüche 1 bis 10, **dadurch gekennzeichnet, dass** das hintere Segment (10) einen Fersenfortsatz aufweist, der sich nach oben erstreckt, ausgelegt, um die hintere Seite der Ferse aufzunehmen.

12. Vorrichtung nach einem der Ansprüche 1 bis 11, **dadurch gekennzeichnet, dass** sie ein oberes Segment (19) umfasst:
- das sich über dem hinteren Segment (10) erstreckt und ein Lager zur Aufnahme des Beins bildet,
- aufweisend:
∘ mindestens einen Abschnitt, der ausgelegt ist, um eine vordere Stütze in Kontakt mit der vorderen Loge des Beins zu bilden, und
∘ mindestens einen Abschnitt, der ausgelegt ist, um eine hintere Stütze in Kontakt mit der hinteren Loge des Beins zu bilden,
wobei das obere Segment (19) elastisch mit dem hinteren Segment (10) verbunden ist, um
- das hintere Segment (10) in seiner Ruheposition zu halten,
- eine relative Verschiebung des oberen Segments (19) und des hinteren Segments mit Bezug aufeinander zu ermöglichen und das obere Segment (19) und das hintere Segment (10) elastisch in die Ruheposition rückzusetzen.

13. Vorrichtung nach einem der Ansprüche 1 bis 12, **dadurch gekennzeichnet, dass** sie mindestens aus einem Verbundmaterial gebildet ist, umfassend mindestens eine Verstärkung in Form von Fasern und mindestens ein Polymermaterial, in dem sich die Fasern erstrecken.

14. Schuh, **dadurch gekennzeichnet, dass** er mit einer orthopädischen Vorrichtung nach einem der vorhergehenden Ansprüche ausgestattet ist.

15. Prothese von mindestens einem Abschnitt einer menschlichen unteren Gliedmaße, **dadurch gekennzeichnet, dass** sie mit einer orthopädischen Vorrichtung nach einem der Ansprüche 1 bis 13 ausgestattet ist.

## Claims

1. Orthopaedic device for a lower limb of a human comprising:
- a front segment (2) adapted to receive the sole of a forefoot of a foot,
- a rear segment (10) adapted to receive the lower face of the heel of said foot,
in which:
- the front segment (2) and the rear segment (10) are formed from two pieces which are distinct from one another,
- the front segment (2) and the rear segment (10) are connected by at least one upper arch (8),
which:
∘ extends above the front segment (2) and the rear segment (10), providing a recess for receiving the foot,
∘ is adapted to keep the front segment (2) and the rear segment (10) in an inactive position,
**characterised in that**:
the upper arch is resilient and adapted to make possible a relative displacement of said front segment (2) and said rear segment (10) in relation to one another, starting from the inactive position, and to bring back the front segment and the rear segment resiliently into the inactive position.

2. Device according to Claim 1, **characterised in that** the front segment (2) is formed from a first plate and the rear segment (10) is formed from a second plate which is distinct from the first plate.

3. Device according to either of Claims 1 or 2, **characterised in that** the front segment (2) and the rear segment (10) extend in the longitudinal prolongation of one another and are longitudinally spaced apart from one another, at least in the inactive position.

4. Device according to Claim 3, **characterised in that** the upper arch is adapted to make possible a relative displacement of said front segment (2) and said rear segment (10) in relation to one another, starting from the inactive position, in a sagittal plane.

5. Device according to one of Claims 1 to 4, **characterised in that** the front segment (2) is adapted to receive the big toe of the foot.

6. Device according to one of Claims 1 to 5, **characterised in that** the upper arch (8) extends between an outer lateral end of said front segment (2) and an inner lateral end of said rear segment (10).

7. Device according to one of Claims 1 to 6, **characterised in that** the front segment (2) extends transversely between an inner lateral edge which is adapted to receive the big toe and an outer lateral edge which is adapted to receive the fifth metatarsus of the foot.

8. Device according to one of Claims 1 to 7, **characterised in that** the rear segment (10) extends transversely between an inner lateral end and an outer lateral border (12).

9. Device according to one of Claims 1 to 8, **characterised in that** the upper arch (8) has an inner appendage (14) which extends laterally and downwards and is disposed longitudinally between the front and the rear segments in such a way as to form a support for the arch of the foot.

10. Device according to one of Claims 1 to 9, **characterised in that** the front segment (2) has an inner lateral border (6) which is adapted to support the big toe of the foot laterally.

11. Device according to one of Claims 1 to 10, **characterised in that** the rear segment (10) comprises a heel appendage which extends upwards and is adapted to receive the rear face of the heel.

12. Device according to one of Claims 1 to 11, **characterised in that** it comprises an upper segment (19) which:
- extends above said rear segment (10), providing a recess for receiving the leg,
- has:
∘ at least one portion which is adapted to form a front support which is in contact with the front compartment of said leg, and
∘ at least one portion which is adapted to form a rear support which is in contact with the rear compartment of said leg,
said upper segment (19) being connected resiliently to said rear segment (10) in such a way as to:
- keep the rear segment (10) in the inactive position,
- make possible a relative displacement of said upper segment (19) and said rear segment in relation to one another, and to bring back the upper segment (19) and the rear segment (10) resiliently into the inactive position.

13. Device according to one of Claims 1 to 12, **characterised in that** it is formed from at least one composite material comprising at least one reinforcement in the form of fibres and at least one polymeric material within which said fibres extend.

14. Shoe **characterised in that** it is equipped with an orthopaedic device according to one of preceding claims.

15. Prosthesis for at least one portion of a lower limb of a human, **characterised in that** it is equipped with an orthopaedic device according to one of Claims 1 to 13.
